# EUROPEAN PATENT SPECIFICATION

(11) **EP 4 251 232 B1**
(45) Date of publication and mention of the grant of the patent: **21.08.2024**
(21) Application number: 21819025.4
(22) Date of filing: 24.11.2021
(51) Int. Cl.: A61M 3/02

(54) **PORTABLE BOWEL IRRIGATION SYSTEM**
TRAGBARES DARMSPÜLUNGSSYSTEM
SYSTÈME PORTABLE POUR L'IRRIGATION DU CÔLON

(30) Priority: 24.11.2020 DK PA202070784
(43) Date of publication of application: 04.10.2023
(73) Proprietor: Coloplast A/S, 3050 Humlebaek (DK)
(72) Inventor: ZEUTHEN, Kristoffer, 2100 København Ø (DK); KNOEDLER, Stephanie, 2990 Nivaa (DK); JENSEN, Nina Hoegh, 2200 Copenhagen N (DK); HVID, Niels, 2950 Vedbaek (DK)
(86) International application number: PCT/DK2021/050347
(87) International publication number: WO 2022/111781

(56) References cited:
- EP-B1- 3 481 460
- US-A1- 2008 275 381
- US-A1- 2015 352 275

## Description

A portable system for irrigation of the bowels is provided. The system comprises a container for an irrigation liquid, and an anal probe for insertion into the rectum of a user. In particular, the system comprises a pump cartridge to be connected to a base unit and tubing for controlling the supply of irrigation liquid to the anal probe.

### Background

Control of voluntary bowel functions is frequently limited or absent in patients suffering from certain disabilities, such as spinal injuries, multiple sclerosis, or spina bifida. Such lack of control of voluntary bowel functions typically results in faecal incontinence or intractable constipation, as patients have significantly reduced ability to sense the presence of faeces in the colon terminal part and the rectum and to sense the evacuation stimulus.

It is known to accomplish bowel emptying by irrigation (i.e. flushing) of the bowels via the rectum, by an irrigating fluid, such as tap water or saline, which is provided through an intermittent catheter with a tip which is configured and sized for insertion into the rectum (the rectum being the terminal section of the alimentary canal; from the sigmoid flexure to the anus), where it remains in a fixed position and held by an inflatable element, which may be inflatable by air or by water with a pump. Once the rectum or stoma has been flushed with the irrigating liquid, the inflatable element is removed, and the liquid and faeces can evacuate. Known irrigation systems suffer from being bulky, complicated, and difficult to clean proper after use.

Users of irrigation systems and health care professionals alike would welcome improvements and simplifications in the construct and use of irrigation systems.

EP3481460 B1 discloses a trans-anal irrigation (TAI) device having a pump base unit, an irrigation fluid reservoir, a wireless electronic controller, fluid tubing, and a disposable rectal catheter in fluid communication with the tubing.

### Summary

The present disclosure provides aspects of a portable irrigation system according to the appended claims.

### Brief Description of the Drawings

The accompanying drawings are included to provide a further understanding of embodiments and are incorporated in and constitute a part of this specification. The figures illustrate embodiments and together with the description serve to explain principles of embodiments. Other embodiments and many of the intended advantages of embodiments will be readily appreciated as they become better understood by reference to the following detailed description. Like reference numerals designate corresponding similar parts.

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention.

In addition, an illustrated embodiment needs not have all the aspects or advantages shown. An aspect or an advantage described in conjunction with a particular embodiment may be practiced in any other embodiments even if not so illustrated, or if not so explicitly described. The scope of the invention defined by the appended claims.
Figure 1 is a perspective view of one embodiment of a portable bowel irrigating system of the invention.
Figure 2 is a more detailed perspective view of a base unit and a pump cartridge of the system according to one embodiment of the invention.
Figure 3 is an exploded, perspective view illustrating components of one embodiment of the irrigation system in a non-assembled condition.
Figure 4 is a schematic, hydraulic diagram applying to different embodiments of a portable irrigation system.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration. It is to be understood that other embodiments may be utilized, and structural or logical changes may be made without departing from the scope of the present invention. The scope of the present invention is defined by the appended claims.

It is to be understood that the features of the various exemplary embodiments described herein may be combined with each other, unless specifically noted otherwise.

Throughout this disclosure, the solid and liquid wastes from the bowels may be referred to as both "output," "waste(s)," and "fluids" interchangeably. A subject using the irrigation system may be referred to as "patient" or "user". However, in some cases "user" may also relate or refer to a health care professional (HCP), such as a doctor/physician, nurse, or others. In those cases, it will either be explicitly stated, or be implicit from the context that the "user" is not the "patient" him- or herself.

The use of the phrase "substantially" as a qualifier to certain features or effects in this disclosure is intended to simply mean that any deviations are within tolerances that would normally be expected by the skilled person in the relevant field.

In the present disclosure a "cartridge" is to be understood as a module designed to be inserted into a larger piece of equipment (in this case into the base unit of the irrigation system).

In the present disclosure, "portable" is to be understood as something that is easy to carry or move, so that it can be used in different places, i.e. is easily and conveniently transported. Particularly, this should be understood is relation to some types of available irrigation systems for the bowels of a user, which are large and bulky and intended for bedridden or hospitalized users only.

In one aspect, the present disclosure relates to a portable system for irrigating the bowels of a user, comprising
a base unit;
a liquid container for an irrigation liquid, the container configured to be removably connected to the base unit;
a pump cartridge comprising an irrigation liquid inlet and a pump mechanism and configured to be removably attachable in the base unit;
an anal probe;
a first tubing configured to provide at least a portion of an irrigation liquid flow path from the pump cartridge towards the anal probe;
wherein the base unit comprises a power supply configured to actuate the pump mechanism and a passage for allowing irrigation liquid from the liquid container to enter the pump mechanism of the pump cartridge through the irrigation liquid inlet of the pump cartridge.

In one implementation, the base unit is configured to be self-supporting and adapted to provide an individual element of the irrigation system. In embodiments, the base unit is configured to be able to stand by itself on a generally level surface of the surroundings. In embodiments, the base unit includes a housing. The base unit housing is configured to hold one or more components of the irrigation system. The one or more components can be held inside the housing and/or entirely or partially on an outer surface of the housing. Some components can be held inside the housing and other components can be held entirely or partially on the outside of the housing. In embodiments, the one or more components of the irrigation system can extend from inside the housing to outside the housing through a wall of the housing. In embodiments, a surface of an outer wall of the base unit housing is configured to receive a liquid container of the irrigation system. In embodiments, the base unit housing includes connectable injection moulded top and bottom portions. In embodiments, a top, or upwardly facing, wall surface of the base unit is configured to receive a liquid container of the irrigation system. In embodiments, the top wall surface of the base unit is shaped or formed such as to receive and provide support for the liquid container. In embodiments, the liquid container includes means for or is shaped to fit with a shape or form of the base unit, such as a shape of a top wall surface of the base unit. In embodiments, a shape of a bottom portion of the liquid container is complementary to a shape of a top surface of the base unit. Thereby, the liquid container and the base unit can combine to carry and hold the liquid container containing an irrigation liquid, thus making the system simple and intuitive and less bulky than known systems. In embodiments, complimentary shapes of the top wall surface of the base unit and the liquid container are generally rounded and adapted for abutment against each other.

In embodiments, the base unit includes means for removably attaching the liquid container to the base unit. The means keep the liquid container in secure connection with the base unit during irrigation. In embodiments, the means is/are located on an outer surface of the housing of the base unit. In embodiments, the means include(s) one or more protrusions extending out from an outer surface of the housing, such as out from a top wall surface of the housing of the base unit. In other embodiments, protrusions can be provided on an outer surface of the liquid container and adapted to engage with suitable receiving means (e.g. a notch or other indentation) on the base unit. Other suitable complementary means for removable attachment of the liquid container in secure connection with the base unit are acceptable. It is noted that the connecting means can also be provided on other surfaces of the base unit and the liquid container than the ones mentioned above.

The base unit includes a power supply configured to actuate the pump mechanism. The power supply is provided in the housing of the base unit and is a permanent component of the irrigation system. In embodiments, the power supply is battery driven. In embodiments, the power supply is driven by power from an external source, such as the mains. In embodiments, the mains provide power for a rechargeable battery pack of the system provided in the base unit which can be charged through a charging socket in the base unit.

The base unit includes a passage for allowing irrigation liquid from the liquid container to enter the pump mechanism of the pump cartridge through the irrigation liquid inlet of the pump cartridge. Thus, the irrigation liquid is supplied in a downstream direction from the liquid container, through the passage in the base unit, into and through the irrigation liquid inlet of the pump cartridge to the pump mechanism, the latter serving to eject (by pumping out) the irrigation liquid from the pump cartridge towards the anal probe via the first tubing. In embodiments, the passage in the base unit for the passage of irrigation liquid is configured to receive an irrigation liquid outlet guide of the liquid container and allow it to extend through the passage and into the irrigation liquid inlet of the pump cartridge.

The pump cartridge is configured to be removably attachable in the base unit. In embodiments, the pump cartridge is configured to be attachable and/or removed or replaced without the use of tools. To these ends, in embodiments, the pump cartridge can include one or more integrated attachment means for easy attachment of the pump cartridge to the base unit. The means can include, but are not limited to, clasp or buckle mechanisms, e.g. a spring actuated clasp. Alternatively, a snap locking mechanism and/or bayonet locking means can be used. According to the invention, these and other features of the pump cartridge provides for the pump cartridge to be replaceable. According to the invention, the removably attachable pump cartridge including the pump mechanism, but provided separately from the power supply in the base unit, provides a bowel irrigation system wherein there is no contact between the irrigation liquid and the drive mechanism of the pumping means. This advantageously prevents contamination of the drive mechanism of the pumping means and further offers a solution wherein the moving parts of the pump in contact with the irrigation liquid, provided in the pump cartridge, can be replaced separately without requiring substitution of the entire pump drive.

In embodiments, the base unit includes a socket or slot configured to receive the pump cartridge of the irrigation system. In embodiments, the socket is located closer to a bottom wall of the base unit than to a top wall of the base unit. In embodiments, the socket of the base unit includes one or more guides adapted to steer and securely receive and hold the pump cartridge of the irrigation system within the socket. In one implementation, the one or more guides in the socket of the base unit include(s) one or more rails or bars. The one or more rails or bars can extend from one or more of a bottom-, top-, end- or sidewall into an internal space of the base unit to form the socket of the base unit. In embodiments, the base unit includes an internal structure, e.g. made from polymer material and/or by injection moulding, which is configured to form the socket of the base unit and receive the pump cartridge. The internal structure of the base unit is also configured to provide support for other components of the irrigation system, including the power supply and/or battery box. In embodiments, an entrance to the socket or slot includes an opening in a sidewall of the housing of the base unit. In embodiments, the pump cartridge includes a flange portion. In embodiments, the flange portion includes an outwardly facing surface of the pump cartridge which is configured to be substantially flush or level with a sidewall of the base unit, in the situation wherein the pump cartridge is inserted in the socket of the base unit.

In embodiments, the pump cartridge includes a casing. In embodiments, the casing is formed from suitable polymer materials and/or lightweight metal materials and/or combinations thereof. In embodiments, the casing includes five walls. In embodiments, the casing includes six walls. In embodiments, the flange portion is configured to form one wall of the casing of the pump cartridge, such as an outwardly facing surface or portion of the casing.

In embodiments, the pump cartridge includes means for engaging with the one or more guides in the socket of the base unit. In embodiments, the means include(s) one or more tracks configured to engage with the one or more rails or bars of the socket. In embodiments, the one or more tracks can be provided integral with one or more walls of the casing of the pump cartridge, such as integral with at least an external surface of the one or more walls of the casing. Alternatively, the one or more tracks can be attached to an outer surface of the casing of the pump cartridge. In embodiments, the socket of the base unit and the pump cartridge can include other suitable complementary means for engaging with each other. In embodiments, the casing of the pump cartridge (and/or the engagement means to engage with the socket) includes a shaft or axle opening configured to receive an axle or similar of an actuator provided in the base unit.

The pump cartridge includes an irrigation liquid inlet. The irrigation liquid inlet is provided in a wall of the casing of the pump cartridge. In embodiments, the irrigation liquid inlet is provided in a top wall of the casing of the pump cartridge. In this, and in other embodiments, the irrigation liquid inlet is configured to substantially align axially with the irrigation liquid passage in the base unit when it is inserted in the socket of the base unit. In embodiments, the liquid passage in the base unit is provided in a position immediately above and adjacent the irrigation liquid inlet in the top wall of the casing of the pump cartridge. In embodiments, gravity facilitates the supply of irrigation liquid from the liquid container to the pump mechanism. In other embodiments, a forced or actuated supply of irrigation liquid from the liquid container can alternatively or additionally be incorporated in the system. In embodiments, the irrigation liquid inlet in the pump cartridge comprises a check (or one-way) valve.

In embodiments, the liquid container includes an irrigation liquid outlet guide. In embodiments, the irrigation liquid outlet guide of the liquid container is provided in a bottom wall (downwardly facing wall when the liquid container is in an upright position) of the liquid container. In embodiments, a pump cartridge check valve includes a valve pushrod (or similar) configured to engage with the irrigation liquid outlet guide of the liquid container, such that the check valve in the pump cartridge can be brought to an open position when the liquid outlet guide of the liquid container engages with (contacts and/or pushes on) the check valve pushrod.

In embodiments, the opening and closing of the check valve are controllable via control means provided with the base unit. In embodiments, the system includes a check valve and a further separate valve allowing to close off the irrigation flow. In embodiments, the base unit includes a control device configured to control the irrigation system. The control device can be configured to include control of the opening and closing of the check valve of the pump cartridge. In embodiments, the control device is an electronic control device.

Further alternatively, or additionally, the irrigation liquid outlet guide in the liquid container can include a check valve. This alternative or additional check valve can similarly be controlled by the control device of the base unit.

Further alternatively, or additionally, in embodiments the irrigation liquid passage in the base unit includes a check valve. This further alternative or additional check valve can similarly be controlled by the control device of the base unit.

In embodiments, the electronic control device includes a processing unit (CPU), which is programmed (or programmable) to control the irrigation system, such as, but not necessarily, controlling an entire irrigation cycle of the system. More than one CPU can be incorporated in the irrigation system. In embodiments, the system includes a remote-control unit configured to communicate wirelessly with the base unit to allow controlling of the irrigation system. In embodiments, the irrigation system can be controlled by the CPU provided in the control device of the base unit or by the CPU in the remote-control unit. Alternatively, the CPUs combine in a joint controlling of the irrigation system.

The pump mechanism of the pump cartridge can be provided by different types of pumping options. The pumping means can be provided by a reciprocating or rotary pumping mechanism. In embodiments, the pump mechanism includes a gear pump. In other embodiments, the pump mechanism includes a peristaltic pump. Other types of pumping mechanisms can be acceptable provided they are suitable for the portable irrigation system according to the invention. In embodiments, gravity is sufficient to provide a priming pressure when the liquid container is located on top of the base unit. In embodiments, a pump vacuum requirement of the pump mechanism is between 5-20 mbar lift, preferably approximately 10 mbar lift. Requiring such a pump vacuum can help provide a certain "pull" pressure to overcome potential surface (or other) tension and thereby avoid any "dry" pump starts.

The components of the irrigation system, including the pump cartridge (with the pump mechanism), should be suitable for handling tap water with a temperature of between 5 and 60 degrees centigrade. The components of the irrigation system should allow a flow within the levels of approximately 1000 mL/min at 0 mbar and 250 mL/min at 100 mbar. Some or all the components of the irrigation system are preferably configured for a bi-directional flow with low pulsation. The components of the irrigation system are adapted for a duty cycle of at least 5 minutes continuous running time per 24 hours. The irrigation system should preferably have a dry run capability sufficient to de-air and dry run at the end of an irrigation cycle of at least 1 minute. In embodiments, the pump cartridge is configured to fulfil a service life of at least 20 hours or 300 L running time. In embodiments, the pump cartridge is configured to fulfil a service life of up to 120 hours or 1500 L running time. Alternatively, or additionally, the pump cartridge is configured to fulfil a service life of at least 2 years shelf time and 1-year usage time.

In embodiments, the pump cartridge defines a first portion of the irrigation liquid flow path extending from the pump cartridge towards the anal probe. In embodiments, the first portion of the irrigation liquid flow path is provided by a first tubing. In embodiments, the first tubing extends through an opening in a wall of the casing of the pump cartridge. In embodiments, the first tubing extends through an opening in the flange portion of the pump cartridge. In embodiments, a minor portion of the first tubing extends from an exit of the pump mechanism inside the pump cartridge and a major portion of the first tubing extends externally of the pump cartridge. In embodiments, one or more of the second tubing and possibly further tubing can extend from inside the pump cartridge to outside the pump cartridge in a similar manner as the first tubing. In embodiments, pump cartridges (in some cases designated pump heads) currently available on markets and suitable and adaptable for use together with the components of the irrigation system include the CAPIOX^{®} SP Disposable Centrifugal Pump, or the Sarns^{™} Centrifugal Disposable Pump, both available from the company Terumo Cardiovascular of Ann Arbor, Michigan, United States. Other suitable, marketed pump cartridges (pump heads) can be acceptable.

In embodiments, the system includes a pressure regulator in the irrigation liquid flow path between the pump cartridge and the anal probe. The pressure regulator allows for regulation of the pressure in the system. The pressure regulator allows for regulation of the pressure with which the irrigation liquid enters the bowels of the user when it exits from the anal probe. In embodiments, the pressure regulator is an automatic pressure regulator. In embodiments, the pressure regulator is a manually controllable pressure regulator. In embodiments, the pressure regulator includes at least one automatic security valve. In embodiments, a pressure sensor measuring the pressure in the irrigation liquid flow path is configured to signal to the security valve in the event of liquid pressure in the irrigation liquid flow rising above a set threshold value. In embodiments, a pressure sensor is configured to measure the pressure in both the irrigation liquid flow path and in the second flow path (for an inflatable balloon). In embodiments, the security valve is provided together with the pressure regulator in a housing of the pressure regulator. In embodiments, the pressure regulator housing includes a spring-actuated lock mechanism and a button for releasably connecting the pressure regulator to tubing or to a tubing adapter. In other embodiments, the pressure sensor and the security valve can be provided in another portion of the irrigation liquid flow path. In some embodiments, the security valve can be a manual security valve. In embodiments, the security valve is a weeping valve.

In embodiments, the first tubing extends from the pump cartridge to the pressure regulator provided in the irrigation liquid flow path between the pump cartridge and the anal probe. In embodiments, the first tubing extends from a liquid exit of the pump mechanism (located inside the casing of the pump cartridge) to the pressure regulator provided in the irrigation liquid flow path between the pump cartridge and the anal probe. In another embodiment, the first tubing extends from a position at an opening in a wall of the casing of the pump cartridge (but not from a position inside the casing) to the pressure regulator provided in the irrigation liquid flow path between the pump cartridge and the anal probe. In the context of the present invention, it is to be understood that the irrigation system can include further tubing, such as, but not limited to, a second, third, and fourth tubing or even further tubing. Moreover, in this disclosure, the term "towards the anal probe" is to be understood as meaning in a downstream flow direction wherein the irrigation liquid exits from the pump mechanism and flows in the direction of the anal probe through the first tubing, and, in embodiments, additionally through a second and potentially further tubing.

In embodiments, the irrigation system includes an expandable retention element provided on or with the anal probe. The expandable retention element is adapted to be expanded after the anal probe has been inserted into the rectum of a user to retain the anal probe in the rectum during the irrigation cycle of the system. [claim 20] In embodiments, the irrigation system includes a second flow path for supplying and withdrawing liquid to and from an inflatable balloon provided on the anal probe. The inflatable balloon on the anal probe then provides the expandable retention element and is expanded by pumping liquid into it for retaining the anal probe in the rectum of the user for the duration of the irrigation cycle. When the irrigation cycle is completed, the inflatable balloon can be collapsed to a deflated state such that the anal probe can be withdrawn from the rectum. The inflatable balloon is adapted to receive liquid for inflation thereof from the liquid in the liquid container through the second flow path. The inflatable balloon is deflated by reversing the flow of liquid in the second flow path. In embodiments, the second flow path includes a separate tube, i.e. a separate, dedicated "balloon tube" extending from the pump cartridge to the anal probe. It is therefore to be understood that in embodiments, the irrigation liquid is configured to fulfil the dual purposes of supplying irrigation liquid to irrigate the bowels, and to provide liquid to inflate the inflatable balloon on the anal probe to retain it in the rectum during the irrigation cycle.

In embodiments, the single tubing includes a double-lumen tube, wherein one lumen forms at least a portion of the first irrigation liquid flow path for the liquid for irrigating the bowels of the user, and the second lumen forms at least a portion of the second flow path for the liquid for inflating the balloon on the anal probe. In embodiments, the first irrigation liquid flow path includes a check valve (one-way valve) to prevent back-flow of the irrigation liquid used for irrigating the bowels of the user. The second flow path does not include a check valve (a one-way valve) because it provides for bi-directional flow of the (irrigation) liquid used for expanding the inflatable balloon on the anal probe. The second flow path can, however, include a separate valve allowing to close off the liquid flow (to the balloon) in the second flow path. [claim 17] In embodiments, the first tubing includes a double-lumen tube.

In accordance with the invention, the irrigation system is adapted to offer the user increased flexibility during use of an irrigation system as well as to offer increased convenience to the user. One factor in that respect is to reduce complexity of the irritation system and to minimize the requirements for cleaning, drying and other maintenance of the irrigation system. Thus, in implementations, some components of the present irrigation system are configured for single-use, some are configured for multiple-uses (but not permanent use) and some are configured for permanent use. "Permanent" is to be understood as "throughout the lifetime" of the irrigation system. In embodiments, particularly the base unit including the power supply is configured as a permanent component of the irrigation system. In embodiments, particularly the anal probe is configured as a single-use component. In embodiments, particularly the liquid container, the tubing(s), and the pump cartridge are configured as multiple-use components. In embodiments, the tubing(s) and the pump cartridge are provided are provided as one integrated component configured for multiple uses (and replaceable as a whole). In embodiments, the tubing(s), the pump cartridge, and the pressure regulator are provided as one integrated component configured for multiple uses (and replaceable as a whole). In other embodiments, the tubing(s) is/are configured as single-use component(s). In embodiments, the tubing(s) and the anal probe are provided as one integrated single-use component. In embodiments, the pressure regulator is configured as a single-use component. In embodiments, the pump cartridge includes fastening means, such as, but not limited to, one or more screws provided for temporary fastening of the pump cartridge to the base unit. In such embodiments, the pump cartridge is a multiple-use component and can therefore suitably be temporarily fastened to the base unit when inserted in the socket of the base unit. In this respect, "temporary" and "temporarily" is to be understood as ranging from only one use of the system to repeated uses over a period of about 12 months. In embodiments, the pump cartridge is designed to have a lifetime of at least six months from first use.

One advantage of providing a replaceable pump cartridge according to the invention is that the moving parts of the pump mechanism can be substituted, in turn allowing for less costly materials to be used in the construction of the pump mechanism (e.g. plastic material gears). Moreover, a replaceable pump cartridge allows for a high standard of cleanliness in that the pump cartridge can be replaced in the event of bacteria and/or other contaminants forming on the surfaces of those portions of the cartridge being in contact with the irrigation liquid, or for other reasons. Similar effects can be achieved in embodiments wherein other components of the irrigation system, including the tubing(s) and/or the pressure regulator, are provided as one integrated component. Further advantageously, the irrigation system of the present invention provides a system solution without any electrical connections/engagement between the component holding the irrigation liquid, i.e. the liquid container, and the base unit. Moreover, in the present invention, the irrigation liquid does not engage with the power supply of the base unit, and there is no possibility of electrical current passing between the irrigation liquid and the pump actuating component of the system. This construction reduces complexity and is increases security, reliability, and easy drying and cleaning of the irrigation system.

In embodiments, the base unit includes one (and only one) control button. In embodiments, the base unit includes one control button configured to communicate with the control device of the irrigation system. The control button can be arranged substantially at level with an outer surface of the housing of the base unit. In embodiments, the control button is provided in a sidewall of the housing of the base unit. In embodiments, the control button is configured to communicate electronically with the control device. In embodiments, the control device is programmed to initiate different program cycles or different cycle segments in response to different numbers of engagement of the (one and only one) control button by a user (i.e. one push of the button triggers one type of action, two consecutive pushes trigger another type of action etc.). Alternatively, the control button can be configured to be in mechanical engagement with one or more components or mechanisms of the irrigation system such as, but not limited to, the control device, the check valve(s) described above, the power supply, and the pump mechanism of the pump cartridge.

The irrigation system includes a liquid container for an irrigation liquid. The irrigation liquid can be temperate tap water or in some cases a saline physiological solution. The irrigation liquid container is configured to be removably connected to the base unit. In embodiments, the liquid container includes one or more means configured to engage with the means for removably attaching the liquid container in secure connection provided on the base unit. In embodiments, the liquid container includes one or more oblong indentations configured to engage with the one or more protrusions extending from the outer surface of the housing of the base unit.

In embodiments, the irrigation liquid outlet guide of the liquid container, which is provided in a bottom wall of the liquid container, includes a piece of pipe formed sealingly around (encircling) an opening in the bottom wall of the liquid container and adapted to extend into the irrigation liquid passage in the base unit. In embodiments, the piece of pipe is adapted to extend through the liquid passage in the base unit and into the irrigation liquid inlet of the pump cartridge. In embodiments, the piece of pipe is relatively rigid and made from polymeric material ('rigid' being relative to a polymeric material of the walls of the liquid container). In embodiments, the relatively rigid piece of pipe is configured to engage with a spring-actuated check valve of the irrigation liquid inlet of the pump cartridge. The engagement causes the check valve to open and allow irrigation liquid to enter from the liquid container into the pump mechanism of the pump cartridge when the liquid container is engaged with the base unit. One advantage of providing a piece of pipe extending into engagement with the irrigation liquid inlet (and/or with the check valve opening) of the pump cartridge, is that contact between the irrigation liquid and the base unit can be entirely excluded, thus providing a "dry" connection between the liquid container and the base unit of the irrigation system, and thereby avoiding the need for drying and cleaning of the base unit. Further, this also avoids contact between the irrigation liquid and the components inside the base unit, particularly the power supply for actuating the pump mechanism.

The base unit includes a power supply configured to actuate the pump mechanism. In embodiments, the power supply comprises at least one electric motor. The electric motor can be powered by one or more batteries or by supply of electricity from an external source. The base unit can comprise a transformer for transforming the voltage of an external source into a low voltage for use in the system. In embodiments, the base unit includes two power supplies configured to actuate two or more individual parts of the pump mechanism. If suitable, the base unit can include more than two power supplies, such as more than two electric motors.

In embodiments, the power supply includes at least one shaft configured to engage with the pump mechanism through a casing of the pump cartridge and configured to actuate the pump mechanism. In embodiments, a portion of the shaft includes a gear wheel which is configured to engage with a gear wheel of the pump mechanism inside the casing such that the pump mechanism is actuated when the power supply makes the shaft rotate. Other suitable engagement solutions between the shaft and the pump mechanism are also acceptable.

In embodiments, the first tubing extends between the pump cartridge and the pressure regulator, and the system further comprises a second tubing that extends between the pressure regulator and the anal probe such that the first tubing, the pressure regulator, and the second tubing, combine to form the irrigation liquid flow path from the pump cartridge to the anal probe. In other words, the irrigation liquid flow path includes at least the first tubing and the second tubing and the pressure regulator. In embodiments, the irrigation liquid flow path includes additional tubing and/or additional components, such as, but not limited to, the anal probe and/or the pump cartridge.

In embodiments, the irrigation system comprises a second tubing extending from the pressure regulator to the anal probe, i.e. downstream of the pressure regulator in the direction of flow of the irrigation liquid. In embodiments, a check valve is provided in the second tubing. The check valve in the second tubing prevents reflux of irrigation liquid form the anal probe to the pressure regulator (i.e. upstream), thereby preventing any unintended and undesired contamination of the pressure regulator and/or other upstream components of the irrigation system (e.g. by incorrect operation). In other embodiments, the check valve is provided at a liquid outlet of the pressure regulator and can be provided integral with the pressure regulator.

In embodiments, the portable irrigation system includes a first pinch valve tube downstream of the pump mechanism. Alternative valve types including knife valves can be applied. A pinch valve tube is to be understood as being the pinch valve and a portion of a tube (or tubing) to which the pinch valve is connected and effects on. In embodiments, the first pinch valve tube is provided with the pump cartridge. In embodiments, the first pinch valve tube is provided within the casing of the pump cartridge. In embodiments, the first pinch valve tube is provided externally of the casing of the pump cartridge. Alternatively, the first pinch valve tube is provided in (or as part of) the first tubing. The first pinch valve tube can be provided in (or as part of) the first tubing between the pump cartridge and the pressure regulator. Particularly, the first pinch valve tube can be provided in a portion of the irrigation liquid flow path being in the first tubing and within the casing of the pump cartridge. In embodiments, the first tubing includes a single-lumen tubing. The first pinch valve is adapted to open to allow for the supply of irrigation liquid to the irrigation liquid flow path from the pump cartridge towards the anal probe. The first pinch valve tube is configured to communicate with the control device of the base unit, the control device configured to signal opening and closing instructions to the first pinch valve.

In embodiments, the portable irrigation system comprises a first pinch valve tube downstream of the pump mechanism and a second pinch valve tube downstream of the pump mechanism. In embodiments, the first pinch valve tube and the second pinch valve tube are provided with the pump cartridge. In embodiments, the first pinch valve tube and the second pinch valve tube are provided within the casing of the pump cartridge. Alternatively, the first pinch valve and the second pinch valve are provided in (or as part of) the first tubing. In embodiments, the first pinch valve and the second pinch valve are provided in (or as part of) the first tubing between the pump cartridge and the pressure regulator. In embodiments, the first pinch valve and the second pinch valve are provided in a portion of the irrigation liquid flow path being in (or as part of) the first tubing and within the casing of the pump cartridge.

In embodiments, the first pinch valve tube is provided in (or as part of) a first lumen of a double-lumen first tubing and the second pinch valve is provided in (or as part of) a second lumen of the double-lumen first tubing. The first pinch valve is adapted to open and close to allow for the supply of irrigation liquid in the irrigation liquid flow path from the pump cartridge towards the anal probe. In embodiments, the second pinch valve is adapted to open and close to allow for supply of liquid to a liquid inflatable balloon provided on the anal probe to retain the anal probe in the rectum of a user during irrigation. Each of the first pinch valve and the second pinch valve is configured to communicate with the control device of the base unit, the control device signalling opening or closing instructions to the respective pinch valve.

In embodiments, the base unit includes at least one valve actuator unit configured to engage with and actuate a pinch valve. In embodiments, the valve actuator unit is an electrical actuator. In embodiments, wherein the first pinch valve tube is provided with or within the pump cartridge, and in embodiments wherein the first pinch valve tube and the second pinch valve tube are provided with or within the pump cartridge, the valve actuator unit can include a shaft or axle configured to engage with the pinch valve. In embodiments, wherein there are more than one pinch valve tube, each pinch valve can be actuated by separate valve actuator units. In embodiments, the irrigation system includes at least two valve actuator units to engage with a first and a second pinch valve tube. In embodiments, wherein the pump cartridge includes a casing, the valve actuator unit can be adapted to engage with the pinch valve tube through a wall of the casing, such as, but not limited to, via a shaft or axle extending from the valve actuator unit to the pinch valve tube through the casing wall. In embodiments, the valve actuator unit is configured to engage the pinch valve tube within the casing through an opening in a sidewall of the casing of the pump cartridge. In embodiments, a shaft or axle of the valve actuator unit is configured for mating engagement with a drive mechanism of the pinch valve tube through an opening in the casing of the pump cartridge. In embodiments, the shaft or axle engages with the pinch valve through an axial movement of the shaft or axle of the valve actuator unit. In embodiments, the shaft or axle engages with the pinch valve through rotational movement of the shaft or axle.

The irrigation system includes an anal probe configured to be inserted into the rectum of a user. In embodiments, the anal probe includes a catheter portion. The catheter portion is suitable for insertion into the rectum of the user and for expelling of the irrigation liquid from the catheter portion. In embodiments, the catheter portion includes a catheter tip for insertion into the rectum of the user and for expelling of the irrigation liquid from the catheter tip. In embodiments, the anal probe includes a catheter portion and a handle portion. In embodiments, the handle portion is a separate component which is configured to be connected to the catheter portion of the anal probe and to the tubing of the system (first or second and/or other tubing). In embodiments, the handle portion is configured to be connected to the catheter portion and the tubing delivering the liquid such that rotation of the handle portion is possible even when connected to one or both other portions. In embodiments, the handle portion is configured to offer full (360 degrees) rotation. In embodiments, this is facilitated by a rotatable hinge connection.

In embodiments, the irrigation system includes a manual separator unit located in the irrigation liquid flow path downstream of the pump cartridge. The manual separator unit can be located to allow for disconnection of at least the irrigation liquid flow path between the pump cartridge and the pressure regulator; or between the pressure regulator and the anal probe. In that manner, the pressure regulator and/or the anal probe and/or the tubing can be exchanged individually and/or cleaned and/or dried individually. In embodiments, the manual separator provides an "emergency stop" capable of immediate disconnection of liquid flow to both the anal probe and the inflatable balloon. In embodiments, multiple manual separator units can be included in the irrigation system. In embodiments, the manual separator unit is releasably connectable to the anal probe. In embodiments, the manual separator unit includes a spring-actuated lock mechanism and a button for its release for providing the releasable connection. In embodiments, the manual separator unit is provided integral with the handle portion of the anal probe, such that they present as integrated component.

In embodiments, the irrigation system includes one or more pressure sensors. The system can include one single pressure sensor to sense the pressure in the irrigation liquid flow path. Alternatively, the system can include a plurality of pressure sensors for detecting the pressure at a plurality of positions in the irrigation system. In embodiments, the system includes at least one pressure sensor per tubing or lumen extending downstream from the pump cartridge. In embodiments, the pressure sensor includes a tubing portion including a short-sectioned bypass channel inside which a pressure in the irrigation liquid flow path or in the second flow path (for the inflatable balloon) can be detected (possibly further provided with a liquid membrane to secure complete "dry" pressure detection). In embodiments, the pressure sensor is a relative pressure sensor (configured to sense a relative pressure) and includes a separate atmospheric pressure sensor in the base unit. Further, in embodiments, the base unit (or the separate atmospheric pressure sensor in the base unit) includes a fluid blocked air inlet port (or canal) in contact with the surroundings for allowing the detection of the surrounding atmospheric pressure.

In embodiments, the pump cartridge includes at least one pressure sensor. In embodiments, the pump cartridge includes one pressure sensor per downstream tubing or lumen extending from the pump cartridge. In embodiments, a pressure sensor is provided in the first tubing. In embodiments, a pressure sensor is provided in the second tubing. In embodiments, a pressure sensor is provided with the pressure regulator.

### Detailed description of the drawings

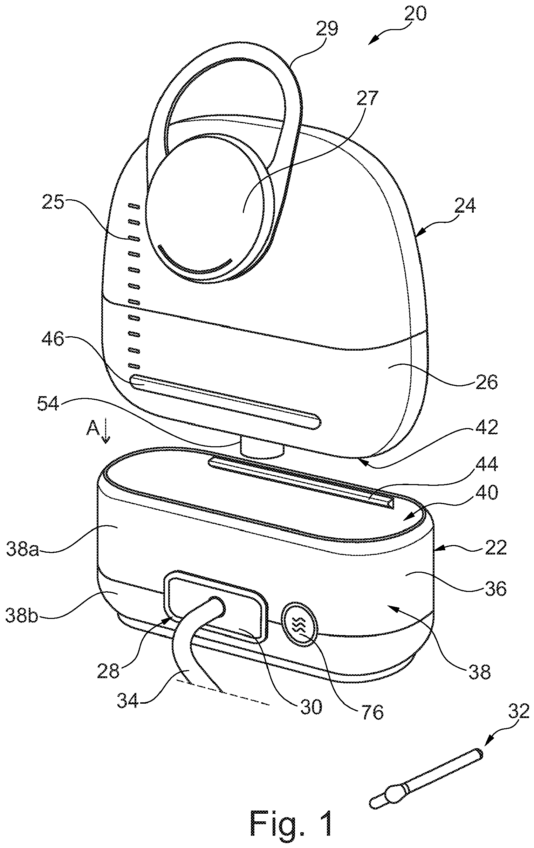

Figure 1 concerns a first aspect of the present invention and is a perspective view illustrating one embodiment of a portable system 20 for irrigating the bowels of a user. The system includes a base unit 22, which is self-supporting and provides one of individual element of the irrigation system 20. In the illustrated embodiment the base unit 22 is adapted to stand on a generally level surface of the surroundings. The system 20 includes another individual element being a liquid container 24 for containing an irrigation liquid 26. In the illustrated view, the liquid container 24 is shown in a position above the base unit 22. It is to be understood that the liquid container 24 is configured to be removably connected to the base unit 22. In the embodiment of Figure 1, the liquid container 24 is shown in a situation where it is about to be connected to the base unit 22 by moving the liquid container 24 in the direction of arrow A.

The system 20 also includes a pump cartridge 28, which in the illustrated view is shown inserted in the base unit 22. The pump cartridge 28 is removably attached to the base unit 22. In Figure 1, only a flange portion 30 forming an external surface of the pump cartridge 28 is visible, as it will be understood that the remainder of the pump cartridge 28 is inserted in the base unit 22.

The system 20 also includes an anal probe 32 to be inserted into the rectum of a user for irrigation of the bowels. In the illustrated embodiment, a first tubing 34 provides at least a portion of an irrigation liquid flow path from the pump cartridge 28 towards the anal probe 32. In Figure 1, the first tubing 34 is shown to be "fading", however it is to be understood that in embodiments the first tubing extends to a connection portion of the anal probe 32.

The base unit 22 includes a housing 36 configured to hold one or more components of the irrigation system 20. The housing 36 includes an outer wall 38. In the illustrated embodiment, a surface of the outer wall 38 of the base unit housing 36 is ready to receive the liquid container 24. Particularly, a top, or upwardly facing, surface 40 of the outer wall of the base unit 22 is ready to receive the liquid container 24. In the illustrated embodiment, the top wall surface 40 of the base unit 22 is shaped to receive and provide support for the liquid container 24 in that a generally rounded shape of a bottom portion 42 (wall) of the liquid container 24 is complementary to the shape of the top wall surface 40 of the base unit 22. In embodiments, complimentary shapes of the top wall surface of the base unit and the liquid container are generally rounded and adapted for abutment against each other.

In the illustrated embodiment, the base unit 22 a first protrusion 44 extending out from a portion of the top wall surface 40 of the housing 38 of the base unit 22. It is to be understood that the top wall surface 40 in the illustrated embodiment is not planar. In this embodiment it has a bowl shape. A second protrusion (not shown) extends out from another portion of the top wall surface 40 across from the first protrusion 44. The protrusions 44 are adapted to engage with indentations 46 on the liquid container 24. In the embodiment of Figure 1, the liquid container 24 includes irrigation liquid volume indicia 25 on a wall surface of the liquid container 24. Further, the liquid container 24 is shown including a closure 27 and a handle portion 29, which can be rotatably provided.

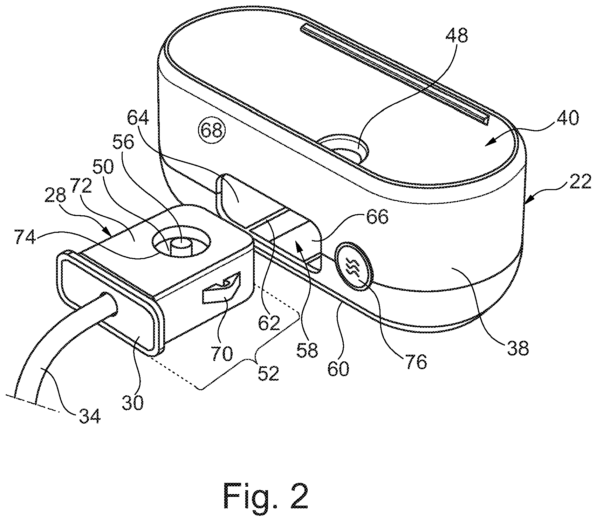

Turning for a moment now to the view of Figure 2 illustrating a perspective view of one embodiment of the base unit 22 and the pump cartridge 28 of the system 20 in more detail. As illustrated in Figure 2, the base unit 22 includes a passage 48 for allowing irrigation liquid 26 from the liquid container 24 (not shown in Fig. 2) to enter the pump mechanism of the pump cartridge through an irrigation liquid inlet 50 of the pump cartridge 28. In this manner, irrigation liquid 26 is supplied in a downstream direction from the liquid container 24, through the passage 48 in the base unit 22, into and through the irrigation liquid inlet 50 of the pump cartridge 28 to the pump mechanism inside a casing 52 of the pump cartridge 28. The pump mechanism serves to pump out the irrigation liquid from the pump cartridge 28 towards the anal probe 32 via the first tubing 34. The passage 48 in the base unit 22 is configured to receive an irrigation liquid outlet guide 54 of the liquid container 24 (Figure 1) and allow it to extend through the passage 48 and into the irrigation liquid inlet 50 of the pump cartridge 28. In the embodiment of Figure 1, the irrigation liquid outlet guide 54 is provided in a bottom wall 42 of the liquid container 24. The irrigation liquid outlet guide 54 of the liquid container 24 is shown to include a piece of pipe formed sealingly around an opening in the bottom wall 42. It is understood that the piece of pipe is adapted to extend into and through the irrigation liquid passage 48 in the base unit 22 and into the irrigation liquid inlet 50 of the pump cartridge 28.

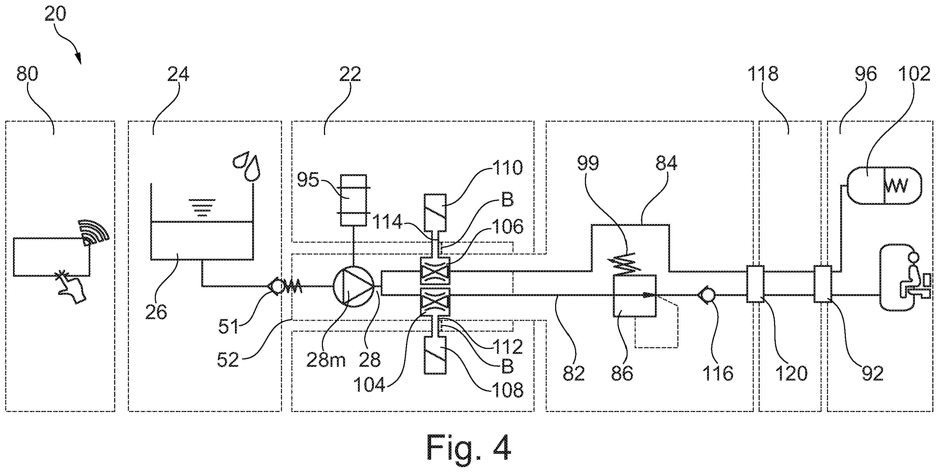

The irrigation liquid inlet 50 in Figure 2 includes a check valve 51 (Fig. 4) of which a stopper surface 74 is visible in the irrigation liquid inlet 50. The check valve (and the stopper surface) can be spring actuated. When the relatively rigid piece of pipe of the liquid outlet guide 54 engages with the spring-actuated check valve stopper surface 74, it exerts a downward force either on the stopper surface 74 or on a valve pushrod 56 to open the check valve in the liquid inlet 50 and allow the flow of irrigation liquid 26 from the liquid container. Thereby, contact between the irrigation liquid 26 and the base unit 22 is avoided, thus providing a "dry" connection between the liquid container 24 and the base unit 22 of the irrigation system.

In Figure 2, the base unit 22 is shown to further include a socket 58 for receiving the pump cartridge 28. The socket 58 is located closer to a bottom wall 60 than to a top wall 40 of the base unit 22. In the illustrated embodiment, the socket 58 includes one or more guides in the form of rails 62 adapted to steer and securely receive and hold the pump cartridge 28 within the socket 58. The rails 62 are shown to extend from the bottom wall 60 into an internal space of the base unit 22 forming the socket 58 of the base unit. In Figure 2, the base unit 22 includes an injection moulded internal structure 64 including the socket 58. The internal structure 64 of the base unit is also configured to provide support for other components of the irrigation system 20, including a power supply and/or battery box (not shown in Fig.2). In Figure 2, an entrance to the socket 58 is formed by an opening 66 in a sidewall 68 of the housing 38 of the base unit.

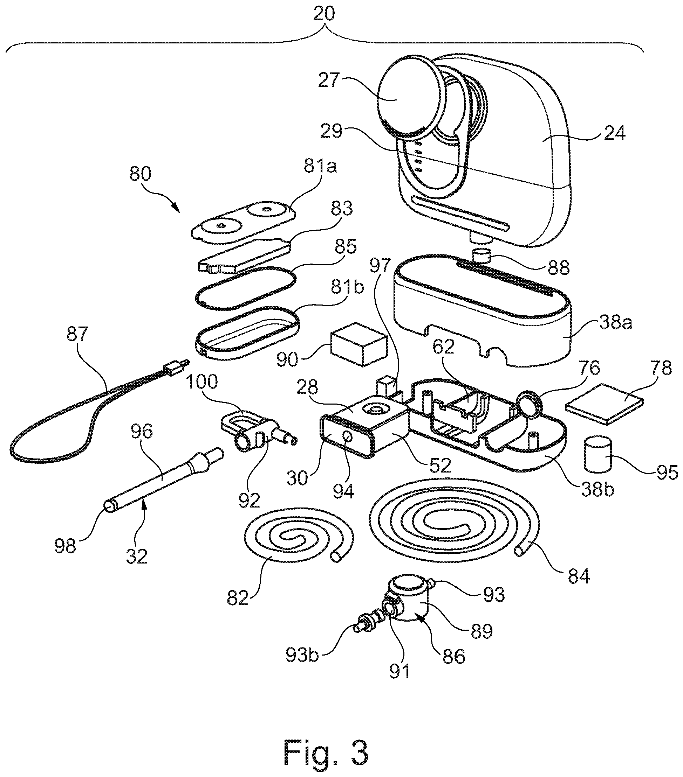

In the embodiment of Figures 1 and 2, the base unit 22 includes one (and only one) control button 76, which is configured to communicate with a control device 78 (Fig. 3) of the irrigation system. The control button 76 is arranged substantially at level with an outer surface or sidewall 68 of the housing 38 of the base unit 22. The control button 76 is configured to communicate with the control device 78, which is programmed to initiate different actions in response to different numbers of pushes on the control button.

In the embodiment of Figure 2, the pump cartridge 28 includes flange portion 30. The flange portion 30 forms am outwardly facing wall of the casing 52 of the pump cartridge 28. As shown in the embodiment of Figure 1, the outwardly facing surface of the flange portion 30 is configured to be substantially flush with the sidewall 68 of the base unit when the pump cartridge 28 is inserted in the socket 58 of the base unit 22. The pump cartridge 28 in Figure 2 further includes engagement means 70 on an outer surface of the casing 52 configured to engage with complementary means in the socket 58 of the base unit 22. The means 70 in Figure 2 includes a spring tensioned, movable protrusion on a sidewall of the casing 52.

In the embodiment of Figure 2, the irrigation liquid inlet 50 is provided in a top wall 72 of the casing 52 of the pump cartridge 28. It is understood that in Figure 2 the irrigation liquid inlet 50 is configured to substantially align axially with the irrigation liquid passage 48 in the base unit 22 when the pump cartridge is inserted in the socket 58 of the base unit and is provided in a position immediately above and adjacent the irrigation liquid inlet 50 of the pump cartridge. Gravity facilitates the supply of irrigation liquid 26 from the liquid container 24 to the pump mechanism.

Figure 3 is an exploded, perspective view illustrating components of one embodiment of the irrigation system 20. In Figure 3, the components of the system 20 are shown in a non-assembled condition.

In the embodiment of Figure 3, the base unit 22 includes an electronic control device 78 configured to control the irrigation system 20. The control device is shown to include an integrated circuit. The system 20 also includes a remote-control unit 80 which communicates wirelessly with the control device 78 of the base unit 22 to control the irrigation system. Either the remote-control unit 80 or the control device 78 or both, includes a central processing unit (CPU). The remote-control unit 80 of Figure 3 includes first and second halves 81a, 81b of a housing, an integrated circuit 83 and a support 85. The remote-control unit 80 includes a cord or string 87 for attachment around the wrist of the user or similar.

Figure 3 further illustrates the liquid container 24, the pump cartridge 28, first tubing 82 and second tubing 84, a pressure regulator 86 and the anal probe 32. In the embodiment of Figure 3, the base unit 22 includes a housing top 38a and a housing bottom 38b. A rubber gasket 88 of the irrigation liquid outlet guide 54 is also visible. Further, Figure 3 illustrates a rechargeable battery 90 of the system provided in the base unit 22, a charging socket 97, a power supply 95 of the system and a manual separator unit 92 which is releasably connectable to the anal probe 32 and to a tubing 82, 84. It is understood that at least the rechargeable battery 90, the power supply 95 and the control device 78 are provided with the housing 38 when the system 20 is assembled.

It is to be understood that in Figure 3, the first portion of the irrigation liquid flow path is provided by the first tubing 82, which extends through an opening 94 in the casing 52 of the pump cartridge 28 provided in the flange portion 30 of the pump cartridge 28. A minor portion of the first tubing 82 extends from an exit of the pump mechanism located inside the casing of the pump cartridge 28 (and not shown) through the opening 94 in the flange portion 30, and a major portion of the first tubing 82 extends externally of the pump cartridge 28 to provide the downstream direction irrigation flow path of the system 20 towards the anal probe 32. The system 20 includes a pressure regulator 86 to be inserted in the irrigation liquid flow path between the pump cartridge 28 and the anal probe 32. The pressure regulator 86 includes at least one security valve and/or a pressure sensor (not visible as these are provided in a housing 89 of the pressure regulator 86). With the embodiments of the system 20 illustrated in Figure 3, the first tubing 82 extends from the pump cartridge 28 to an inlet 91 of the pressure regulator 86 and the second tubing 84 extends from an outlet 93 of the pressure regulator 86 to the anal probe 32, thereby combining to provide the irrigation liquid flow path of the system 20. The second tubing 84 extends from the pressure regulator 86 to the anal probe 32, i.e. downstream of the pressure regulator in the direction of flow of the irrigation liquid. A check valve (not shown) provided in the second tubing prevents reflux of irrigation liquid 26 form the anal probe 32 towards the pressure regulator 86 (i.e. upstream). The check valve can also be provided at the outlet 93 of the pressure regulator 86 and integral therewith. In some embodiments, the system includes one or more additional tube connectors, such as the one 93b shown next to the inlet 91 of the pressure regulator 86.

In the embodiment of Figure 3, the irrigation system 20 includes anal probe 32 configured to be inserted into the rectum of a user. The anal probe 32 includes a catheter portion 96 for insertion into the rectum of the user and for expelling of the irrigation liquid 26. The catheter portion 96 includes a catheter tip 98. At least a segment of the catheter portion 96 at an end portion opposite the catheter tip 98 can be tapered. The anal probe 32 further includes a handle portion 100, which is a separate component and connectable to the catheter portion 96 of the anal probe 32 and to the tubing 84, 86 of the system 20. The handle portion 100 is connected to the catheter portion 96 and the tubing 84, 86 such that 360 degrees rotation of the handle portion 100 is possible even when it is connected to one or both.

The irrigation system 20 of Figure 3 includes a manual separator unit 92 to be located in the irrigation liquid flow path downstream of the pump cartridge 28 and allow for disconnection of the irrigation liquid flow path between the pump cartridge 28 and the pressure regulator 86 or between the pressure regulator 86 and the anal probe 32. In Figure 3, the manual separator unit 92 is releasably connectable to the anal probe 32 and is provided integral with the handle portion 100 of the anal probe 32.

The power supply 95 is configured to actuate the pump mechanism of the pump cartridge 28. The power supply 95 is provided in the housing 38 of the base unit 22 and is a permanent component of the irrigation system 20. In the embodiment of Figure 3, the power supply 95 is an electric motor. The power supply 95 in Figure 3 is driven by power from the rechargeable battery 90. The rechargeable battery 90 is charged by power from the mains via a charging socket 97. The base unit 22 can additionally comprise a transformer for transforming the voltage of the external source into a low voltage for use in the electric motor of the system 20.

Figure 4 is a schematic, hydraulic diagram applying to different embodiments of the portable irrigation system 20 according to the invention. The system 20 includes a base unit 22, a liquid container 24, a pump cartridge 28, an anal probe 96 and at least a first tubing 82. The system 20 includes a first pinch valve tube 104 downstream of the pump mechanism 28m of the pump cartridge 28. In the embodiment of Figure 4, the first pinch valve tube 104 is provided with the pump cartridge 28 is provided as part of (in or on) the first tubing 82. In Figure 4, the first pinch valve tube 104 is provided between (downstream of) the pump mechanism 28m and the pressure regulator 86. Thus, the first pinch valve tube 104 is provided in a portion of the irrigation liquid flow path.

In the embodiment of Figure 4, the system 20 also includes a second pinch valve tube 106 downstream of the pump mechanism 28m. Both the first pinch valve tube 104 and the second pinch valve tube 106 are provided with the pump cartridge 28. The first pinch valve tube 104 helps control the delivery of irrigation liquid to the anal probe and the second pinch valve tube 106 helps control a liquid flow for inflation or deflation of the inflatable balloon 102 provided on the anal probe 96. The first pinch valve tube 104 is adapted to open and close to allow for the supply of irrigation liquid 26 in the irrigation liquid flow path from the pump cartridge 28 towards the anal probe 96. The second pinch valve tube 106 is adapted to open and close to allow for supply of liquid to the liquid inflatable balloon 102 provided on the anal probe 96 to retain the anal probe in the rectum of a user during irrigation.

In the embodiment of Figure 4, each of the first pinch valve tube 104 and the second pinch valve tube 106 are provided in separate tubing. The first pinch valve tube 104 is provided in first tubing 82 and the second pinch valve tube 106 is provided in second tubing 84. Both the first pinch valve tube 104 and the second pinch valve tube 106 are provided between (downstream of) the pump mechanism 28m and the pressure regulator 86. It is to be understood that the first tubing and the second tubing can also be provided as a first lumen and a second lumen in a double-lumen (first) tubing. The pressure regulator 86 in Figure 4 includes a security valve 99.

In the embodiments of Figure 4, the base unit 22 includes a first valve actuator unit 108 configured to engage with and actuate the first pinch valve tube 104 and a second valve actuator unit 110 configured to engage with and actuate the second pinch valve tube 1016. The first valve actuator unit 108 includes an axle 112 configured to engage with the first pinch valve tube 104. The second valve actuator unit 110 includes an axle 114 configured to engage with the second pinch valve tube 106. As schematically indicated by double-ended arrows B in Figure 4, in embodiments wherein the pump cartridge 28 includes a casing 52, each of the first valve actuator unit 108 and the second valve actuator unit 110 engages with the respective pinch valve tube 104, 106 through a wall of the casing 52 via axle 112, 114 extending from the valve actuator unit to the pinch valve tube through the casing wall.

In the embodiments of Figure 4, the irrigation system 20 includes an inflatable balloon 102 provided on the anal probe 96, which provides an expandable retention element that can retain the anal probe 96 in the rectum of the user for the duration of an irrigation cycle. When the irrigation cycle is completed, the inflatable balloon 102 is collapsed to a deflated state and the anal probe 96 withdrawn from the rectum. The inflatable balloon 102 is adapted to receive liquid 26 from the liquid container 24 through the second tubing 84. It is therefore understood that in embodiments, the irrigation liquid 26 is configured to fulfil the dual purposes of supplying irrigation liquid to irrigate the bowels, and to provide liquid to inflate the inflatable balloon 102 on the anal probe 96 to retain it in the rectum during the irrigation cycle. However, the first tubing 82 may include a double-lumen tube, wherein one lumen provides the first irrigation liquid flow path for the liquid for irrigating the bowels of the user, and the second lumen forms the second flow path for the liquid for inflating the balloon 102 on the anal probe 96. In the embodiments of Figure 4, the first tubing includes or is connected to a check valve 116 for preventing back-flow of the irrigation liquid used for irrigating the bowels of the user. The second tubing 84 does not include a check valve because it allows bi-directional flow of the liquid for expanding and deflating the inflatable balloon 102.

In the embodiments of Figure 4, the system 20 can optionally include a third (or even further) tubing 118. The third tubing 118 is shown to be connected to the first tubing 82 and the second tubing 84 via a further manual separator unit 120.

The embodied system 20 of Figure 4 includes a remote-control unit 80 configured to communicate wirelessly with the base unit 22 to allow controlling of the irrigation system 20.

In the system overview provided by Figure 4, and in accordance with the detailed description above, the components of the embodiment of the system 20 can be understood to have the following lifetimes: The remote-control unit 80, the liquid container 24 and the base unit 22 are permanent components designed to last throughout the lifetime of the system 20. The pump cartridge 28, the pressure regulator 86 and the tubing 82 and 84 are designed to fulfil a lifetime of about 6-12 months. The tubing between the pressure regulator 86 and the anal probe 96, in the view of Figure 4 this is the third tubing 118, is designed for a lifetime of about 3 months. Finally, the anal probe 96 is designed as a single-use component to be exchanged after each irrigation cycle.

Further, in the system overview provided by Figure 4, and in accordance with the detailed description above, the components of the embodiment of the system 20 can be understood as having the following maintenance requirements: The remote-control unit 80 and the base unit 22 require no maintenance. The liquid container 24 requires occasional maintenance (cleaning/disinfection). The pump cartridge 28, the pressure regulator 86 and the tubing 82, 84 and 118 require only to be drip dried after each irrigation cycle. The anal probe 96 requires no maintenance as it is exchanged after each irrigation cycle.

Although specific embodiments have been illustrated and described herein, it will be appreciated by those of ordinary skill in the art that a variety of alternate and/or equivalent implementations may be substituted for the specific embodiments shown and described. This application is intended to cover any adaptations or variations of body side members for ostomy appliances as discussed herein. Therefore, it is intended that this invention be limited only by the claims.

## Claims

1. A portable bowel irrigating system (20) for irrigating the bowels of a user, comprising:
a base unit (22);
a liquid container (24) for an irrigation liquid, the container configured to be removably connected to the base unit (22);
a pump cartridge (28) comprising an irrigation liquid inlet (50) and a pump mechanism (28m);
an anal probe (96);
a first tubing configured to provide at least a portion of an irrigation liquid flow path from the pump cartridge (28) towards the anal probe (96);
wherein the base unit (22) comprises a power supply (95) configured to actuate the pump mechanism (28m) and a passage for allowing irrigation liquid from the liquid container (24) to enter the pump mechanism (28m) of the pump cartridge (28) through the irrigation liquid inlet (50) of the pump cartridge (28);
wherein the system is **characterised in that** the pump cartridge (28) is configured to be removably attachable to the base unit (22).

2. The system of claim 1, comprising a pressure regulator (86) provided in the irrigation liquid flow path between the pump cartridge (28) and the anal probe (96).

3. The system of claim 2, wherein the first tubing extends between the pump cartridge (28) and the pressure regulator (86), the system (20) further comprising a second tubing that extends between the pressure regulator (86) and the anal probe (96) such that the first tubing, the pressure regulator (86) and the second tubing combine to form the irrigation liquid flow path from the pump cartridge (28) to the anal probe (96).

4. The system of any one of the preceding claims comprising a first pinch valve tube (104) downstream of the pump mechanism.

5. The system of any one of claims 1-4 comprising a first pinch valve tube (104) downstream of the pump mechanism (28m) and a second pinch valve tube (106) downstream of the pump mechanism.

6. The system of claim 4 or 5, wherein the base unit (22) comprises at least one valve actuator unit (108,110) configured to engage with and actuate a pinch valve.

7. The system of any one of the preceding claims, wherein the irrigation liquid inlet (50) in the pump cartridge (28) comprises a check valve (116).

8. The system of any one of claims 1-7, wherein the irrigation liquid passage in the base unit (22) comprises a check valve (116).

9. The system of any one of the preceding claims, wherein the power supply (95) comprises at least one shaft configured to engage with the pump mechanism (28m) through a casing of the pump cartridge (28) and configured to actuate the pump mechanism (28m).

10. The system of claim 1, further comprising a manual separator unit located in the irrigation liquid flow path downstream of the pump cartridge (28).

11. The system of claim 10, wherein the manual separator unit is releasably connectable to the anal probe (96).

12. The system of any one of the preceding claims, wherein the anal probe (96) comprises a catheter portion.

13. The system of any one of the preceding claims, wherein the anal probe (96) comprises a catheter portion and a handle portion (100).

14. The system of any one of the preceding claims, wherein the base unit (22) comprises one control button (76) configured to communicate with a control device (78) of the irrigation system (20).

15. The system of any one of the preceding claims comprising one or more pressure sensors.

16. The system of claim 15, wherein the pump cartridge (28) comprises at least one pressure sensor.

17. The system of any one of the preceding claims, wherein the first tubing comprises a double-lumen tube.

18. The system of any one of the preceding claims, wherein the base unit (22) includes a socket or slot configured to receive the pump cartridge (28).

19. The system of claim 1, wherein the irrigation liquid inlet (50) of the pump cartridge (28) is provided in a top wall of a casing of the pump cartridge (28).

20. The system of any one of the preceding claims, comprising a second flow path for supplying and withdrawing liquid to and from an inflatable balloon (102) provided on the anal probe (96).

21. The system of any one of the preceding claims, further comprising a remote-control configured to communicate wirelessly with the base unit (22) to allow controlling of the irrigation system (20).

## Patentansprüche

1. Tragbares Darmspülungssystem (20) zum Spülen des Darms eines Benutzers, umfassend:
eine Basiseinheit (22);
einen Flüssigkeitsbehälter (24) für eine Spülflüssigkeit, wobei der Behälter ausgestaltet ist, um entfernbar mit der Basiseinheit (22) verbunden zu werden;
eine Pumpkartusche (28), die einen Spülflüssigkeitseinlass (50) und einen Pumpmechanismus (28m) umfasst;
eine Analsonde (96);
einen ersten Schlauch, der ausgestaltet ist, um mindestens einen Anteil eines Spülflüssigkeitsflusspfads von der Pumpkartusche (28) zu der Analsonde (96) bereitzustellen;
wobei die Basiseinheit (22) eine Energieversorgung (95), die ausgestaltet ist, um den Pumpmechanismus (28m) zu betätigen, und einen Durchgang umfasst, um zu ermöglichen, dass Spülflüssigkeit aus dem Flüssigkeitsbehälter (24) in den Pumpmechanismus (28m) der Pumpkartusche (28) durch den Spülflüssigkeitseinlass (50) der Pumpkartusche (28) hindurch eintritt;
wobei das System **dadurch gekennzeichnet ist, dass** die Pumpkartusche (28) ausgestaltet ist, um entfernbar an der Basiseinheit (22) anbringbar zu sein.

2. System nach Anspruch 1, umfassend einen Druckregler (86), der in dem Spülflüssigkeitsflusspfad zwischen der Pumpkartusche (28) und der Analsonde (96) bereitgestellt wird.

3. System nach Anspruch 2, wobei der erste Schlauch sich zwischen der Pumpkartusche (28) und dem Druckregler (86) erstreckt, wobei das System (20) zudem einen zweiten Schlauch umfasst, der sich zwischen dem Druckregler (86) und der Analsonde (96) erstreckt, so dass der erste Schlauch, der Druckregler (86) und der zweite Schlauch kombiniert werden, um den Spülflüssigkeitsflusspfad von der Pumpkartusche (28) zu der Analsonde (96) zu bilden.

4. System nach einem der vorhergehenden Ansprüche, umfassend einen ersten Quetschventilschlauch (104) stromabwärts von dem Pumpmechanismus.

5. System nach einem der Ansprüche 1 bis 4, umfassend einen ersten Quetschventilschlauch (104) stromabwärts von dem Pumpmechanismus (28m) und einen zweiten Quetschventilschlauch (106) stromabwärts von dem Pumpmechanismus.

6. System nach Anspruch 4 oder 5, wobei die Basiseinheit (22) mindestens eine Ventilaktuatoreinheit (108, 110) umfasst, die ausgestaltet ist, um in Eingriff mit einem Quetschventil zu kommen und dieses zu betätigen.

7. System nach einem der vorhergehenden Ansprüche, wobei der Spülflüssigkeitseinlass (50) in der Pumpkartusche (28) ein Sperrventil (116) umfasst.

8. System nach einem der Ansprüche 1 bis 7, wobei der Spülflüssigkeitsdurchgang in der Basiseinheit (22) ein Sperrventil (116) umfasst.

9. System nach einem der vorhergehenden Ansprüche, wobei die Energieversorgung (95) mindestens einen Schaft umfasst, der ausgestaltet ist, um durch eine Umhüllung der Pumpkartusche (28) hindurch in Eingriff mit dem Pumpmechanismus (28m) zu kommen und ausgestaltet ist, um den Pumpmechanismus (28m) zu betätigen.

10. System nach Anspruch 1, des Weiteren umfassend eine manuelle Separatoreinheit, die sich in dem Spülflüssigkeitsflusspfad stromabwärts von der Pumpkartusche (28) befindet.

11. System nach Anspruch 10, wobei die manuelle Separatoreinheit lösbar mit der Analsonde (96) verbindbar ist.

12. System nach einem der vorhergehenden Ansprüche, wobei die Analsonde (96) einen Katheteranteil umfasst.

13. System nach einem der vorhergehenden Ansprüche, wobei die Analsonde (96) einen Katheteranteil und einen Griffanteil (100) umfasst.

14. System nach einem der vorhergehenden Ansprüche, wobei die Basiseinheit (22) einen Steuerknopf (76) umfasst, der ausgestaltet ist, um mit einer Steuervorrichtung (78) des Spülsystems (20) zu kommunizieren.

15. System nach einem der vorhergehenden Ansprüche, umfassend einen oder mehrere Drucksensoren.

16. System nach Anspruch 15, wobei die Pumpkartusche (28) mindestens einen Drucksensor umfasst.

17. System nach einem der vorhergehenden Ansprüche, wobei der erste Schlauch einen doppellumigen Schlauch umfasst.

18. System nach einem der vorhergehenden Ansprüche, wobei die Basiseinheit (22) einen Sockel oder Schlitz einschließt, der ausgestaltet ist, um die Pumpkartusche (28) aufzunehmen.

19. System nach Anspruch 1, wobei der Spülflüssigkeitseinlass (50) der Pumpkartusche (28) in einer Oberwand einer Umhüllung der Pumpkartusche (28) bereitgestellt wird.

20. System nach einem der vorhergehenden Ansprüche, umfassend einen zweiten Flusspfad zum Zuführen und Abziehen von Flüssigkeit zu und von einem befüllbaren Ballon (102), der an der Analsonde (96) bereitgestellt wird.

21. System nach einem der vorhergehenden Ansprüche, des Weiteren umfassend eine Fernbedienung, die ausgestaltet ist, um drahtlos mit der Basiseinheit (22) zu kommunizieren, um Steuerung des Spülsystems (20) zu ermöglichen.

## Revendications

1. Système portable d'irrigation des intestins (20) permettant d'irriguer les intestins d'un utilisateur, comprenant :
une unité de base (22) ;
un récipient de liquide (24) pour un liquide d'irrigation, le récipient étant configuré pour être raccordé de manière amovible à l'unité de base (22) ;
une cartouche de pompe (28) comprenant une entrée de liquide d'irrigation (50) et un mécanisme de pompe (28m) ;
une sonde anale (96) ;
une première tubulure configurée pour fournir au moins une partie d'un chemin d'écoulement de liquide d'irrigation depuis la cartouche de pompe (28) jusqu'à la sonde anale (96) ;
dans lequel l'unité de base (22) comprend une alimentation électrique (95) configurée pour actionner le mécanisme de pompe (28m) et un passage permettant au liquide d'irrigation provenant du récipient de liquide (24) d'entrer dans le mécanisme de pompe (28m) de la cartouche de pompe (28) par l'entrée de liquide d'irrigation (50) de la cartouche de pompe (28) ;
dans lequel le système est **caractérisé en ce que** la cartouche de pompe (28) est configurée pour être fixée de manière amovible à l'unité de base (22).

2. Système selon la revendication 1, comprenant un régulateur de pression (86) fourni dans le chemin d'écoulement de liquide d'irrigation entre la cartouche de pompe (28) et la sonde anale (96).

3. Système selon la revendication 2, dans lequel la première tubulure s'étend entre la cartouche de pompe (28) et le régulateur de pression (86), le système (20) comprenant en outre une seconde tubulure qui s'étend entre le régulateur de pression (86) et la sonde anale (96) de telle sorte que la première tubulure, le régulateur de pression (86) et la seconde tubulure se combinent pour former le chemin d'écoulement de liquide d'irrigation de la cartouche de pompe (28) jusqu'à la sonde anale (96).

4. Système selon l'une quelconque des revendications précédentes comprenant un premier tube de vanne à manchon (104) en aval du mécanisme de pompe.

5. Système selon l'une quelconque des revendications 1 à 4 comprenant un premier tube de vanne à manchon (104) en aval du mécanisme de pompe (28m) et un second tube de vanne à manchon (106) en aval du mécanisme de pompe.

6. Système selon la revendication 4 ou 5, dans lequel l'unité de base (22) comprend au moins une unité d'actionnement de vanne (108, 110) configurée pour entrer en prise avec une vanne à manchon et actionner celle-ci.

7. Système selon l'une quelconque des revendications précédentes, dans lequel l'entrée de liquide d'irrigation (50) dans la cartouche de pompe (28) comprend un clapet antiretour (116).

8. Système selon l'une quelconque des revendications 1 à 7, dans lequel le passage de liquide d'irrigation dans l'unité de base (22) comprend un clapet antiretour (116).

9. Système selon l'une quelconque des revendications précédentes, dans lequel l'alimentation électrique (95) comprend au moins un arbre configuré pour entrer en prise avec le mécanisme de pompe (28m) à travers un boîtier de la cartouche de pompe (28) et configuré pour actionner le mécanisme de pompe (28m).

10. Système selon la revendication 1, comprenant en outre une unité de séparation manuelle située dans le chemin d'écoulement de liquide d'irrigation en aval de la cartouche de pompe (28).

11. Système selon la revendication 10, dans lequel l'unité de séparation manuelle peut être raccordée de manière amovible à la sonde anale (96).

12. Système selon l'une quelconque des revendications précédentes, dans lequel la sonde anale (96) comprend une partie cathéter.

13. Système selon l'une quelconque des revendications précédentes, dans lequel la sonde anale (96) comprend une partie cathéter et une partie poignée (100).

14. Système selon l'une quelconque des revendications précédentes, dans lequel l'unité de base (22) comprend un bouton de commande (76) configuré pour communiquer avec un dispositif de commande (78) du système d'irrigation (20).

15. Système selon l'une quelconque des revendications précédentes comprenant un ou plusieurs capteurs de pression.

16. Système selon la revendication 15, dans lequel la cartouche de pompe (28) comprend au moins un capteur de pression.

17. Système selon l'une quelconque des revendications précédentes, dans lequel la première tubulure comprend un tube à double lumière.

18. Système selon l'une quelconque des revendications précédentes, dans lequel l'unité de base (22) comporte une douille ou une fente configurée pour recevoir la cartouche de pompe (28).

19. Système selon la revendication 1, dans lequel l'entrée de liquide d'irrigation (50) de la cartouche de pompe (28) est fournie dans une paroi supérieure d'un boîtier de la cartouche de pompe (28).

20. Système selon l'une quelconque des revendications précédentes, comprenant un second chemin d'écoulement pour l'alimentation et le retrait du liquide vers et depuis un ballonnet gonflable (102) fourni sur la sonde anale (96).

21. Système selon l'une quelconque des revendications précédentes, comprenant en outre une télécommande configurée pour communiquer sans fil avec l'unité de base (22) afin de permettre de commander le système d'irrigation (20).
